# FASCICULE DE BREVET EUROPEEN

(11) **EP 3 911 238 B1**
(45) Date de publication et mention de la délivrance du brevet: **28.06.2023**
(21) Numéro de dépôt: 20702412.6
(22) Date de dépôt: 14.01.2020
(51) Int. Cl.: A61B 6/03, A61B 6/12, A61B 6/00, G06F 3/01, G02B 27/01, A61B 17/34, G06T 19/00, A61B 34/20, A61B 34/10

(54) **DISPOSITIF MÉDICAL EN RADIOLOGIE INTERVENTIONNELLE DE GUIDAGE EN TEMPS RÉEL D'UNE AIGUILLE MÉDICALE D'OPÉRATION DANS UN VOLUME**
MEDIZINISCHE VORRICHTUNG ZUM EINSATZ IN DER INTERVENTIONELLEN RADIOLOGIE ZUR ECHTZEIT-FÜHRUNG EINER MEDIZINISCHEN OPERATIONSNADEL IN EINEM VOLUMEN
MEDICAL DEVICE IN INTERVENTIONAL RADIOLOGY FOR REAL-TIME GUIDING OF A MEDICAL OPERATING NEEDLE IN A VOLUME

(30) Priorité: 17.01.2019 EP 19020031
(43) Date de publication de la demande: 24.11.2021
(73) Titulaire: Université Paris-Est Créteil Val de Marne, 94010 Creteil Cedex (FR); Assistance Publique-Hôpitaux de Paris, 75004 Paris (FR); Centre national de la recherche scientifique, 75016 Paris (FR); Université Gustave Eiffel, 77420 Champs-Sur-Marne (FR)
(72) Inventeur: TACHER, Vania, 75012 PARIS (FR); PICO, Christopher, 77220 TOURNAN EN BRIE (FR); KOBEITER, Hicham, 75013 PARIS (FR); GRANDPIERRE, Thierry, 93700 DRANCY (FR); SACCENTI, Laetitia, 75011 PARIS (FR)
(74) Mandataire: Demulsant, Xavier
(86) Numéro de dépôt international: PCT/EP2020/050831
(87) Numéro de publication internationale: WO 2020/148292

(56) Documents cités:
- US-A1- 2005 203 380
- US-A1- 2016 324 580
- US-A1- 2017 258 526
- US-A1- 2018 116 732
- US-A1- 2018 303 377

## Description

### DOMAINE DE L'INVENTION

La présente invention concerne un dispositif médical pour un usage en radiologie interventionnelle permettant de guider en temps réel, en continu et en trois dimensions l'avancée d'une aiguille dans un corps humain vers une cible, grâce à la réalité mixte.

### ETAT DE LA TECHNIQUE

Les interventions médicales guidées par l'image sont de plus en plus fréquentes par les avancées technologiques qui concernent à la fois le matériel sophistiqué et les modalités d'imagerie disponibles. Ces interventions peuvent être effectuées par voie endovasculaire ou par voie percutanée et utilisent diverses modalités d'imagerie telles que les rayons X (scanner, ou avec la tomographie dite en anglais « cone-beam computed tomography » (CBCT), fluoroscopie, angiographie soustraite), les ondes acoustiques (échographie), la résonance magnétique (imagerie par résonance magnétique (IRM)) ou encore l'imagerie multimodale (ou fusion d'images issues de différentes modalités d'images citées ci-dessus). Le but des avancées technologiques est de guider ces interventions de façon précise tout en limitant l'usage de produit de contraste iodé ou des rayons X du fait de leurs caractères néfastes pour le sujet (néphrotoxicité et effets stochastiques et déterministes sur les tissus biologiques, respectivement).

Les gestes réalisés par voie percutanée permettent de réaliser des biopsies, des ponctions, des drainages et des ablations. Les gestes percutanés sont fréquemment réalisés par guidage scanner ou CBCT lorsqu'ils concernent des structures intérieures pulmonaires, osseuses, ou digestives profondes (rétropéritonéales ou péritonéales). Les gestes réalisés en scanner présentent pour inconvénient l'usage de rayons X avec leur effet délétère connu sur les tissus biologiques et un angle limité à quelques degrés (0-15°) de la voie d'abord selon les constructeurs. Ceci limite ainsi la possibilité de réaliser certains gestes avec des axes quasi-verticaux et compliquent la possibilité de réaliser des voies d'abord avec des angles plus prononcés.

Le CBCT permet également de réaliser des gestes par voie percutanée et est restreint par un champ de vue limité par rapport au scanner et une qualité d'images moindre. Cependant, les angles des voies d'abord peuvent aller jusqu'à 45°.

L'inconvénient majeur de ces moyens de guidage (scanner et CBCT) est en effet l'usage préalable des rayons X pour guider le geste (imagerie de planification réalisée immédiatement avant le geste) mais également pour réaliser des imageries de contrôle(s) lors de l'avancée progressive de l'aiguille afin de vérifier le bon positionnement de l'aiguille. Ces imageries de contrôle accroissent davantage les temps d'intervention avec des risques de complications inhérentes, de mouvements du sujet et donc d'échec de l'intervention et d'irradiation accrue.

De nouveaux moyens de guidage ont été développés comme le guidage électromagnétique utilisant les images diagnostiques pré-interventions (ex : scanner). Ce type de procédé permet d'avancer l'aiguille en suivant simultanément le trajet de l'aiguille sur deux plans orthogonaux au sein d'un volume d'imagerie médicale issu d'une nouvelle acquisition d'imagerie de planification (ex : scanner) réalisée immédiatement avant le geste. Ceci permet de réduire voire de s'affranchir de l'usage des rayons X nécessaires lors de la progression de l'aiguille (imagerie de contrôle).

Les désavantages du système sont la nécessité d'une part de réaliser une imagerie préalable de planification irradiante par scanner et d'autre part de suivre simultanément sur un même écran (divisé en deux) l'avancée de l'aiguille selon deux plans orthogonaux. Ce suivi simultané sur les deux plans orthogonaux requiert en effet un entraînement et une attention soutenue pour reconstituer mentalement le parcours, l'orientation et la position de l'aiguille.

### INVENTION

La réalité mixte offre de nouveaux horizons en termes de guidage et s'introduit progressivement dans les salles opératoires et de radiologie interventionnelle. Celle-ci est aujourd'hui essentiellement utilisée pour la planification des interventions par la représentation en 3D d'images post-traitées issues d'imagerie médicale de type DICOM d'un sujet. Elle permet également de visualiser directement les images de fluoroscopie dynamique permettant le guidage directement dans le champ de vision de l'opérateur et non sur un écran.

La publication de brevet US2005/203380 A1 divulgue un tel procédé de navigation en réalité augmentée.

La présente invention présente un dispositif médical de radiologie interventionnelle avec réalité mixte, permettant un guidage en temps réel et en 3D d'une aiguille médicale droite pour réaliser une ponction, biopsie, drainage ou ablation, vers au moins une cible réelle dans un volume réel de corps d'un sujet, comprenant :
- des moyens d'imagerie (fichiers DICOM de type scanner, IRM, CBCT, Pet scanner ou IRM, échographie) ;
- un volume reconstruit en 3D du volume réel, à partir d'images médicales préalablement acquises avant l'intervention issues d'images DICOM, incluant le volume reconstruit en 3D avec des structures intérieures d'intérêt et une ou plusieurs cible reconstruite en 3D correspondant à la cible réelle;
- des moyens de stockage du volume reconstruit en 3D, pré-enregistré avant l'intervention, ces moyens de stockage étant reliés (communiquent avec ou sans fil) aux moyens d'imagerie ;
- des moyens de visualisation par réalité mixte pour visualiser le volume reconstruit en 3D dans un référentiel virtuel de référence, destinés à être portés par un opérateur ;

les moyens de visualisation par réalité mixte étant reliés (communiquent avec ou sans fil) à des moyens de calcul qui permettent de fusionner le volume reconstruit en 3D avec le volume réel;
le volume reconstruit en 3D, une fois fusionné, demeurant stable et figé dans l'espace et dans le temps sur le volume réel, la cible reconstruite 3D étant fusionnée avec la cible réelle,
   - une aiguille médicale droite destinée à être manipulée par l'opérateur dans la partie du corps jusqu'à la cible réelle ;
   - des moyens de localisation et d'orientation en 3D par ondes électromagnétiques, en temps réel, de l'aiguille médicale droite dans la partie du corps, reliés à l'aiguille médicale par un seul émetteur situé à la base de l'aiguille ; donnant sa position par rapport à un récepteur et dont la position est connue ;
   - les moyens de calcul communiquant avec les moyens d'imagerie médicale, les moyens de stockage, les moyens de visualisation, et les moyens de localisation et d'orientation en 3D,
et recevant les coordonnées des moyens de visualisation dans le repère réel, et le positionnement et l'orientation en 3D de l'aiguille médicale droite dans le référentiel réel,
   les moyens de calcul étant configurés pour :
   i) déterminer les déplacements et rotations en temps réel des moyens de visualisation (8) dans le référentiel réel,
   ii) déterminer la position du volume réel dans le référentiel réel,
   iii) créer une aiguille virtuelle droite fusionnée avec l'aiguille médicale réelle lors d'une étape de fusion, sur leur longueur, forme et positionnement, en tenant compte :
      - du recalage en temps réel (synchronisation automatique avec correction manuelle possible) entre le référentiel virtuel de référence et le référentiel réel qui est fonction des déplacements et rotations en temps réel des moyens de visualisation dans le référentiel réel,
      - du positionnement de l'aiguille médicale droite dans le référentiel réel et transmis aux moyens de calcul, par les moyens de localisation et d'orientation en 3D
      - de la forme et de la longueur de l'aiguille médicale droite intégrés dans les moyens de calcul,
afin de visualiser en temps réel à travers les moyens de visualisation, lors de l'intervention, le positionnement et le déplacement en 3D restant à parcourir jusqu'à la cible reconstruite 3D de l'aiguille virtuelle droite dans le volume reconstruit en 3D préenregistré ;
le positionnement virtuel et le déplacement virtuel fusionnant en temps réel à travers le moyens de visualisation, avec le positionnement réel et le déplacement en 3D réel de l'aiguille médicale droite hors et dans le corps du sujet,
pour guider en temps réel le déplacement par l'opérateur de l'aiguille médicale droite dans le volume réel du corps vers la cible réelle, jusqu'à ce que l'extrémité de l'aiguille médicale droite, atteigne la cible réelle du volume réel,
en suivant le déplacement en 3D de l'aiguille virtuelle droite restant à parcourir jusqu'à la cible reconstruite 3D, dans le volume reconstruit en 3D préenregistré et fusionné sur le volume réel.
   structures intérieures

Le dispositif des inventeurs permet de visualiser de façon virtuelle le sujet et ses organes internes extraits d'imagerie médicale préalablement acquise et traitée, et d'y associer un objet comme une aiguille, elle-même guidée (ou localisée) en trois-dimensions et en temps réel par exemple par des ondes électromagnétiques.

L'opérateur peut ainsi guider son aiguille jusqu'à atteindre une cible dans le corps humain avec une vision directe du sujet, ses organes, la cible et l'aiguille par la réalité mixte. Le principe est de réaliser une intervention à l'aide d'une projection virtuelle des organes du sujet qui sont invisibles de l'extérieur dans le monde réel en raison de la peau, des tissus, des muscles etc.. , du corps humain, mais rendu visible par la réalité mixte.

Cette technique permettrait de s'affranchir ou du moins de réduire l'usage des rayons X et de faciliter tout type de geste percutané.

### EXPOSE DE L'INVENTION

Dans une réalisation, le dispositif médical selon l'invention présente :
- une aiguille qui doit être insérée dans le corps d'un sujet humain afin qu'elle atteigne une cible ;
- un émetteur électromagnétique fixé sur la base de l'aiguille ;
- des moyens d'imagerie médicale ;
- un casque/lunette de réalité mixte qui affiche en 3D :
   1) les objets 3D (organes, tissus) obtenus par traitement des images DICOM (scanner, IRM ou CBCT) préalablement acquises et traitées du sujet et recalées avec la position réelle de celui-ci ;
   2) l'aiguille virtuelle
- un récepteur électromagnétique local posé sur la table du sujet ;
- une interface utilisateur visualisée dans les lunettes de réalité mixte qui permet de fusionner préalablement le sujet avec son propre volume post-traité et d'associer en temps réel le positionnement et l'orientation spatiale de l'aiguille par le système électromagnétique.

Le système comprend une interface qui permet de configurer les caractéristiques de l'aiguille (ou autre outils) comme sa longueur et son diamètre et son apparence virtuelle.

### DESCRIPTION DES FIGURES

D'autres objectifs, caractéristiques et avantages sortiront de la description détaillée qui suit en référence aux dessins donnés à titre illustratif et non limitatif parmi lesquels :
- les figures 1A à 1F représentent le principe de l'invention ;
- la figure 2 représente l'aiguille virtuelle avec des indications de couleur (et de longueur) ;
- la figure 3 représente le casque avec les moyens de calcul et de stockage ;
- les figures 4A à 4G représentent une illustration d'application du prototype pour une biopsie d'un nodule hépatique chez un être humain.

### DESCRIPTION GENERALE DE L'INVENTION

Le but de l'invention est de permettre à l'opérateur de guider en trois dimensions - nommé par la suite 3D- et en temps réel une aiguille médicale 1, 1A dans le corps 2 d'un sujet lors d'une séance de radiologie interventionnelle, par réalité mixte.

La réalité mixte est une projection d'objets virtuels représentant des objets réels.

La réalité mixte est une réalité augmentée.

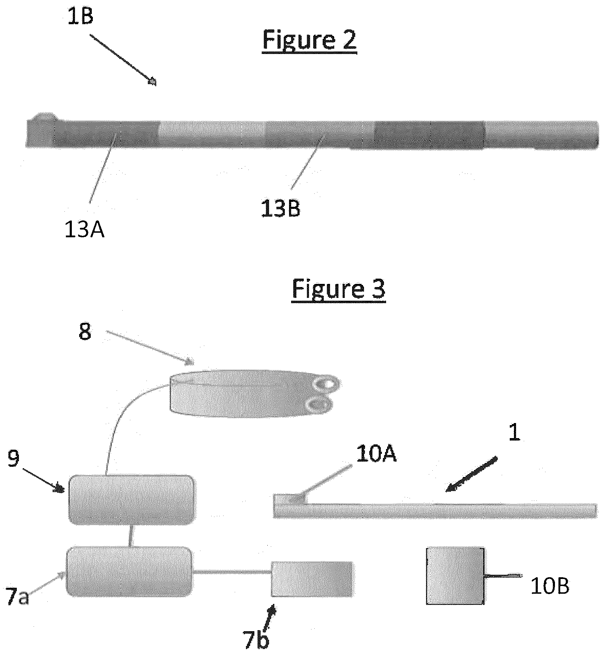

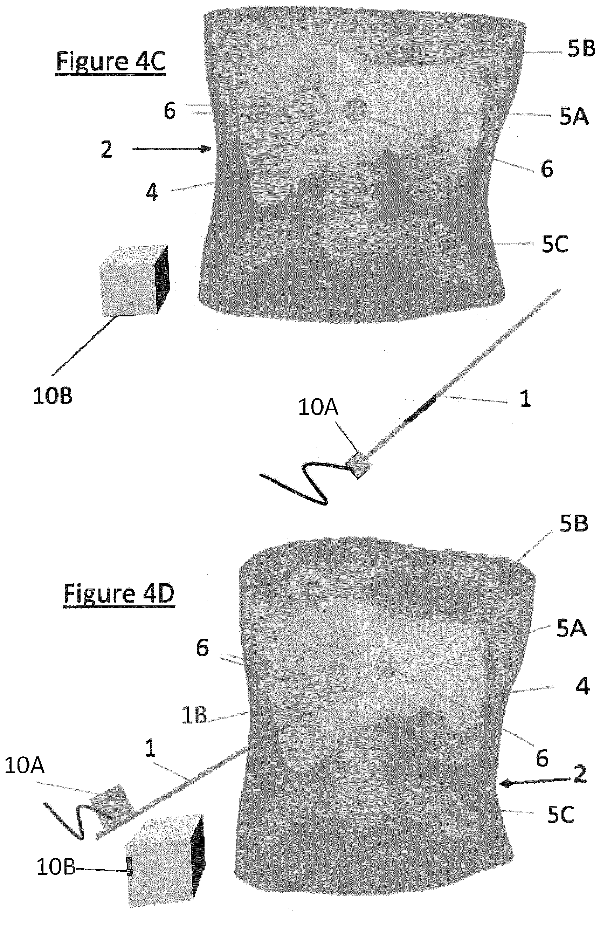

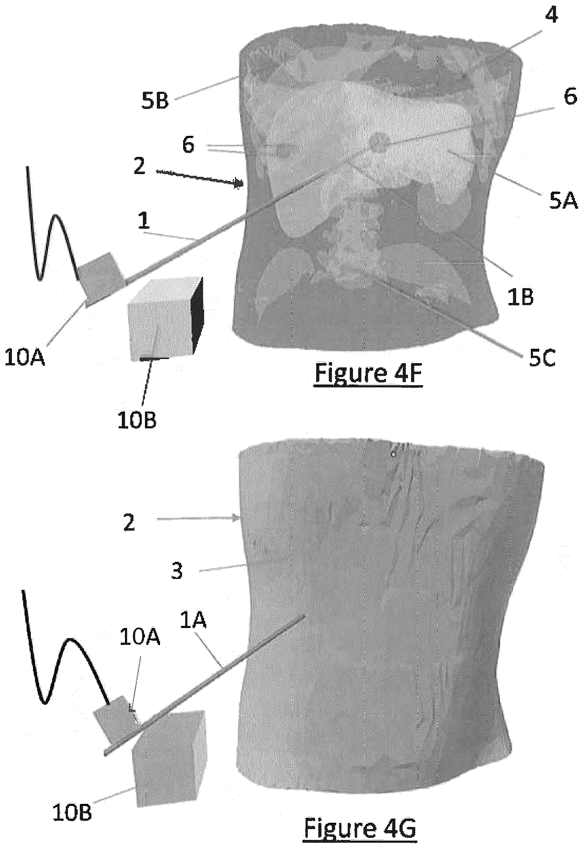

Sur les figures, par souci de simplification,
- l'aiguille médicale a été représentée par le numéro « 1 » quand elle est fusionnée avec l'aiguille virtuelle (vision avec les moyens de visualisation par réalité mixte ; figures 4C, 4D, 4F) et par le numéro « 1A » quand elle est seule (vision sans les moyens de visualisation par réalité mixte ; figure 4G) ;
- l'aiguille virtuelle a été représentée par le numéro « 1 » quand elle est fusionnée avec l'aiguille médicale (vision avec les moyens de visualisation par réalité mixte ; figures 4C, 4D, 4F) et par le numéro « 1B » quand elle est la seule visible avec le moyen de visualisation par réalité mixte (figures 2, 4D, 4F, et même si elle est demeure fusionnée dans la réalité à l'aiguille médicale qui n'est pas visible).

Le dispositif médical de radiologie interventionnelle avec réalité mixte, selon l'invention, permet un guidage en temps réel et 3D d'une aiguille médicale 1,1A vers au moins une cible 6 réelle dans un volume réel 3 de corps 2 d'un sujet.

La cible qui est représentée sur l'ensemble des figures est la cible virtuelle, mais pour des raisons de simplification, comme elle coïncide visuellement pour l'opérateur avec la cible réelle (grâce au dispositif selon l'invention), il a été choisi de mettre le même numéro 6.

Il présente :
- un volume 4 reconstruit en 3D du volume réel 3, à partir d'images médicales préalablement acquises avant l'intervention, par des moyens d'imagerie 7a ;
le volume 4 reconstruit en 3D présentant des structures intérieures d'intérêt 5A, 5B, 5C (exemple organes) et une cible 6 reconstruite 3D correspondant à la cible 6 réelle;
- des moyens de stockage 7b du volume 4 reconstruit en 3D, pré-enregistré avant l'intervention ;
- des moyens de visualisation 8 par réalité mixte pour visualiser le volume 4 reconstruit en 3D dans un référentiel virtuel de référence, destinés à être portés par un opérateur ;

l es moyens de visualisation 8 par réalité mixte étant reliés (communiquent avec ou sans fil) à des moyens de calcul 9 qui permettent de fusionner le volume 4 reconstruit en 3D avec le volume réel 3;
l e volume 4 reconstruit en 3D, une fois fusionné, demeurant stable dans l'espace et dans le temps sur le volume réel 3, la cible 6 reconstruite 3D étant fusionnée avec la cible 6 réelle.

Les moyens de visualisation 8 par réalité mixte peuvent être par exemple un casque ou des lunettes de réalité mixte.

Le casque ou les lunettes de réalité mixte possèdent une interface logicielle qui permet de charger le volume 3D du sujet reconstruit par post traitement à partir d'images DICOM (acronyme en anglais de « Digital imaging and COmmunications in Médiane »). Les images DICOM peuvent être issues d'images de scanner, IRM, CBCT. Les images sont post-traitées afin de construire une représentation tridimensionnelle de ce qui a été acquis. Le post traitement consiste en une phase de sélection et de segmentation des organes à reconstruire, puis une phase de reconstruction volumique à partir de ces segmentations. Enfin, une phase de mise en correspondance entre le volume 3D virtuel du sujet reconstruit et le sujet réel est nécessaire. Cette phase de fusion est appelée « recalage sujet réel - sujet virtuel ».

En d'autres termes, dans l'invention, les dimensions des objets virtuels sont identiques, par les lunettes/casque et la projection oculaire, à celles des objets réels.

Le dispositif médical présente également :
- des moyens 10A, 10B de localisation et d'orientation en 3D en temps réel de l'aiguille médicale 1,1A dans la partie du corps 2, reliés à l'aiguille médicale 1,1A, dans un référentiel réel ;
- les moyens de calcul 9 recevant les coordonnées du moyen de visualisation 8 par réalité mixte dans le repère réel, et reliés (communiquent avec ou sans fil) aux moyens 10A, 10b de localisation et d'orientation en 3D de façon à recevoir le positionnement et l'orientation en 3D de l'aiguille médicale 1,1A dans le référentiel réel.

Les moyens de calcul 9 sont configurés pour :
i) déterminer les déplacements et rotations en temps réel des moyens de visualisation 8 dans le référentiel réel,
ii) déterminer la position du volume réel 3 dans le référentiel réel,
iii) créer une aiguille virtuelle 1,1B fusionnée avec l'aiguille médicale 1,1A réelle en tenant compte :
   - du recalage en temps réel entre le référentiel virtuel de référence et le référentiel réel qui est fonction des déplacements et rotations en temps réel des moyens de visualisation 8 dans le référentiel réel
   - du positionnement de l'aiguille médicale 1,1A dans le référentiel réel et transmis aux moyens de calcul 9,
   - de la forme et de la longueur de l'aiguille médicale 1,1A intégrés dans les moyens de calcul 9.

Les moyens de calcul 9 permettent de visualiser en temps réel via les moyens de visualisation 8, lors de l'intervention, le positionnement et le déplacement en 3D jusqu'à la cible 6 reconstruite 3D de l'aiguille virtuelle 1,1B dans le volume 4 reconstruit en 3D préenregistré ;
le positionnement virtuel et le déplacement virtuel fusionnant à travers les moyens de visualisation 8 avec le positionnement réel et le déplacement en 3D réel de l'aiguille médicale 1,1A dans le corps 2 du sujet, pour guider le déplacement par l'opérateur de l'aiguille médicale 1,1A dans le volume réel 3 vers la cible 6 réelle.

Avantageusement, les moyens de calcul 9 déterminent automatiquement le recalage en temps réel entre le référentiel virtuel de référence et le référentiel réel,
les moyens de calcul 9 ayant été configurés pour créer:
   - un premier objet virtuel à sélectionner dans le référentiel virtuel de référence et dont les coordonnées sont acquises ;
   - un second objet virtuel à sélectionner dans le référentiel virtuel de référence et dont les coordonnées sont acquises,
afin de calculer le décalage entre les deux référentiels,
et donc faire fusionner en permanence et en temps réel aiguille réelle 1, 1A et aiguille virtuelle 1,1B lors du recalage automatique.

Le dispositif médical peut présenter des moyens permettant de calculer le décalage entre les référentiels virtuel et réel, tels que :
- une imagerie pré-intervention (par scanner, IRM, CBCT, échographie, Pet scanner ou Pet-IRM) ;
- des QR codes métalliques disposés sur le sujet, destinés à être imagés en même temps que le sujet avec les moyens d'imagerie et reconstruits dans le référentiel virtuel,
- la surface de peau du sujet reconstruite dans le volume 4 reconstruit en 3D ; exemple il peut être pris la surface de peau du pubis, sternum, nombril, épaules, etc..

Par exemple, les moyens de calcul permettent de calculer le décalage entre les référentiels virtuel et réel, par des objets virtuels choisis par exemple parmi la liste suivante :
- des reliefs cutanés (ombilic, mamelons) ou osseux palpables (côtes, crêtes iliaques, épaule, sternum, épines vertébrales...),
- la surface de la peau, reconstruite en 3D à partir de l'imagerie pré-opératoire.

Les moyens d'imagerie sont aptes à réaliser des images en début ou en cours d'intervention par scanner, CBCT, échographie, de façon à permettre le recalage avec les images médicales préalablement acquises avant l'intervention qui présentent
les volumes d'intérêts reconstruits, dont la cible ; afin de pallier à un éventuel défaut de recalage induit par un mouvement, une déformation (sous la contrainte de l'aiguille) du corps du patient.

Les moyens de calcul 9 peuvent être configurés pour réaliser un second repère virtuel fixe dans le référentiel virtuel de référence à proximité de l'aiguille virtuelle 1,1B pour quantifier le déplacement de l'aiguille virtuelle 1,1B.

Les moyens 10A, 10B de localisation et d'orientation en 3D présentent :
- au moins un capteur de position 10A émettant des ondes électromagnétiques fixé sur l'aiguille médicale 1,1A ;
- un dispositif de réception 10B des ondes électromagnétiques.
Il est à tout fait possible de mettre l'émetteur d'ondes électromagnétiques sur l'aiguille et le récepteur d'ondes électromagnétiques en dehors de l'aiguille sur un endroit de la table

Avantageusement, l'aiguille médicale 1,1A est apte à être utilisée lors de biopsies, ponctions, drainages et ablations pour tout type de cibles du corps 2 humain (exemples : cutanées, mammaires, osseuses, thoraciques, abdominales ou vasculaires artériels et veineux).

Les images médicales utilisées sont de type DICOM (ex : scanner, IRM et CBCT) et sont traitées (ex : segmentation, seuillage, modélisation en volume 3D).

Avantageusement, le volume 4 reconstruit en 3D présente une partie reconstruite comme la surface du sujet (exemple : la peau), des organes d'intérêt et la ou les cible (s).

Le dispositif médical peut comporter des seconds moyens 10A, 10B de localisation et d'orientation en 3D en temps réel, de tout ou partie du sujet pour déterminer sa position et son orientation dans le référentiel réel, et les moyens de calcul communiquent à ces seconds moyens et sont configurés pour recalculer en continu les mouvements de l'aiguille afin d'assurer un recalage en continu entre les référentiels virtuel et réel.

Avantageusement, l'aiguille virtuelle 1,1B peut présenter des repères visibles comme une échelle avec des segments 13A, 13B (exemple: code couleur) permettant d'indiquer la longueur d'aiguille virtuelle 1,1B.

Dans une autre réalisation, l'aiguille virtuelle 1,1B peut présenter uniquement un contour qui coïncide avec le contour de l'aiguille réelle dans la réalité mixte.

Les moyens de calcul 9 peuvent être reliés à des moyens de signalisation sonore pour indiquer par un signal sonore à l'opérateur la distance qui sépare l'aiguille médicale droite 1,1A des structures intérieures d'intérêt 5A, 5B, 5C et des cibles 6. Ce signal sonore peut présenter une intensité ou une fréquence proportionnelle à cette distance ou tout autre type d'information : message etc..

On peut aussi faire en sorte que la couleur des objets modélisés change en fonction de la proximité de l'aiguille : plus l'aiguille approche d'un organe plus celui-ci devient rouge vif etc.

On peut aussi afficher en toute lettres dans le casque/lunettes de réalité mixte la distance entre l'aiguille et l'objet le plus proche. Cette valeur pourrait aussi être affichée sous la forme d'une barre horizontale

Ces moyens de signalisation sonore peuvent être reliés aux moyens de visualisation.

### DESCRIPTION DETAILLEE D'UN MODE DE REALISATION DE L'INVENTION

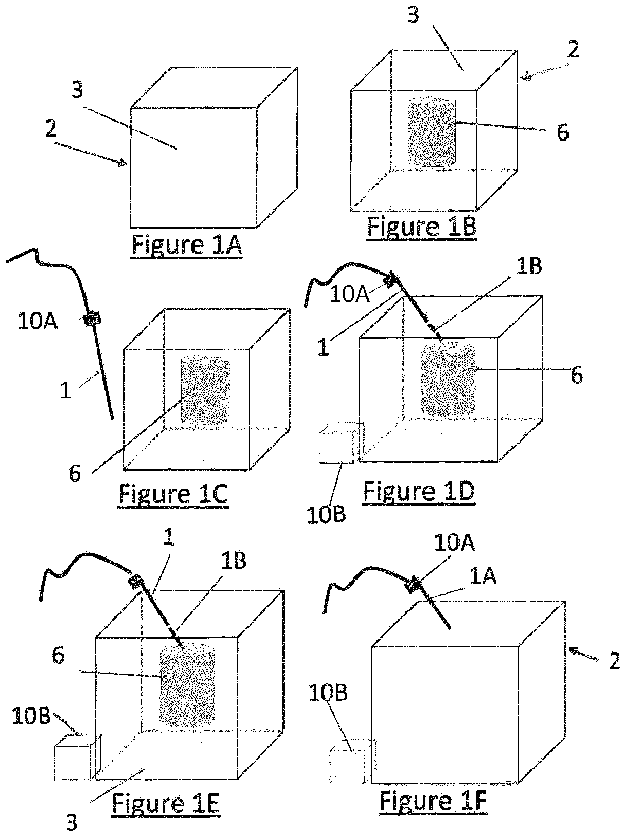

La Figure 1 A représente la vision réelle du sujet de l'invention (ici représenté par un cube), et la figure 1 B représente une vue mixte du sujet et de sa cible au travers de lunettes/casque de réalité mixte. Il est ainsi possible de voir au travers le sujet et d'y apercevoir la cible et sa localisation (i.e. il est possible de voir à travers la peau et les organes).

La figure 1C représente une vue mixte du sujet, de sa cible ainsi que de l'aiguille sur laquelle est fixée l'émetteur électromagnétique, lui-même étant relié au récepteur électromagnétique.

La figure 1D représente une vue mixte du sujet et de sa cible ainsi que de l'aiguille qui progresse dans le sujet pour atteinte la cible. Le mouvement de l'aiguille est visualisé avec son pendant virtuel en 3D et en temps réel.

La figure 1E représente une vue mixte du sujet et de sa cible ainsi que de l'aiguille qui a atteint la cible.

La figure 1F représente la vision réelle du sujet et de l'aiguille introduite et ayant atteint sa cible (sans moyen de visualisation 8 de réalité mixte).

Pendant l'intervention, l'opérateur utilise une aiguille. Sur la base de cette aiguille est fixé un émetteur d'ondes électromagnétiques qui permet de connaître son positionnement et son orientation dans l'espace. Cette aiguille, dont les caractéristiques (longueur, diamètre) ont été prédéfinies par le biais de l'interface utilisateur du casque/lunettes de réalité mixte, nécessite d'être préalablement fusionnée avec son pendant virtuel. Cette phase de fusion est appelée « recalage aiguille virtuelle 1,1B - aiguille réelle 1, 1A ».

Le système de localisation peut utiliser toute sorte de méthode de localisation (ondes électromagnétiques, optiques, mécanique).

Dans le cas d'une localisation électromagnétique, un boitier récepteur d'ondes électromagnétiques est fixé sur la table, proche de la cible.

L'aiguille peut être insérée à n'importe quelle position et suivre un quelconque angle selon le choix de l'opérateur.

L'opérateur avance progressivement l'aiguille grâce au guidage par réalité mixte de la position de l'aiguille jusqu'à atteindre sa cible (Figure 1D). La position et l'orientation de l'aiguille sont obtenues en temps réel par le système de localisation électromagnétique. A partir de ces informations, l'aiguille virtuelle 1,1B (préalablement fusionnée avec l'aiguille réelle) est visible entièrement et en permanence grâce à la réalité mixte, même si l'aiguille réelle n'est plus complètement visible puisque dans le corps 2 du sujet.

Les mains de l'opérateur sont ainsi libres, et son regard est augmenté et orienté vers le sujet et sa cible. Le volume reconstruit est fusionné et calé de façon statique sur le corps 2 du sujet. L'ensemble du corps 2 du sujet et sa projection augmentée de ses organes sont ainsi fusionnés et visibles sur son champ de vision lors de l'avancée de l'aiguille et l'opérateur n'a pas besoin d'écran pour se guider.

Création de l'aiguille virtuelle 1,1B illustrée sur la figure 2:
L'aiguille virtuelle 1,1B est un objet virtuel permettant de représenter virtuellement l'aiguille réelle afin de pouvoir suivre le déplacement de l'aiguille virtuelle 1,1B dans le volume 3D. L'aiguille virtuelle 1,1B est composée de 2 éléments :
- La base, qui représente la position du capteur 10A par rapport à l'émetteur 10B dans l'espace du casque ;
- Le corps multicolore représente le corps de l'aiguille, chaque couleur représente par exemple 5cm sur le corps réel (la taille et l'apparence est configurable par l'interface). Le contrôle de l'aiguille se fait via le récepteur du capteur de position 3D.

Comme indiqué précédemment, la base de l'aiguille est l'endroit où se situe le capteur 10a, il doit être dans la bonne position pour que le réel corresponde au virtuel, c'est le rôle de l'étape de « recalage aiguille virtuelle 1,1B - aiguille réelle » vu précédemment.

La sélection d'un modèle à charger se fait via une interface graphique affichée en réalité mixte. Le modèle 3D, une fois chargé est fusionné avec le corps 2 du sujet dans le monde réel via un processus de calibration manuelle obtenue grâce à la superposition du modèle 3D et du corps 2 du sujet. Ce processus de calibration est appelé « recalage sujet virtuel - sujet réel ». Il est réalisé à l'aide de l'interface graphique qui affiche des boutons virtuels mais il peut aussi être réalisé à l'aide d'une manette de jeux connectée au casque ou à l'aide d'un clavier sans fil connecté au casque.

L'objectif est d'étalonner maintenant les repères du casque virtuel, du monde réel et des aiguilles réelle et virtuelle.

Dans un premier temps, il est nécessaire de faire coïncider les repères virtuels du casque et ceux de l'aiguille réelle et virtuelle.

Le recalage « aiguille virtuelle 1,1B - aiguille réelle » peut être réalisé de deux façons.

Soit manuellement à l'aide de l'interface graphique (ou d'une manette de jeux ou d'un clavier sans fil connecté au casque).

Soit semi-automatiquement : on fait apparaître des cubes virtuels à des positions 3D connues par le système. L'utilisateur doit alors amener la base de l'aiguille virtuelle 1,1B (pour le moment décalée de l'aiguille réelle puisque c'est le but de cette étape de les fusionner) tour à tour sur chacun de ces cubes. Un algorithme calcul ensuite automatiquement le décalage existant afin de le compenser et ainsi faire coïncider l'aiguille virtuelle 1,1B et l'aiguille réelle.

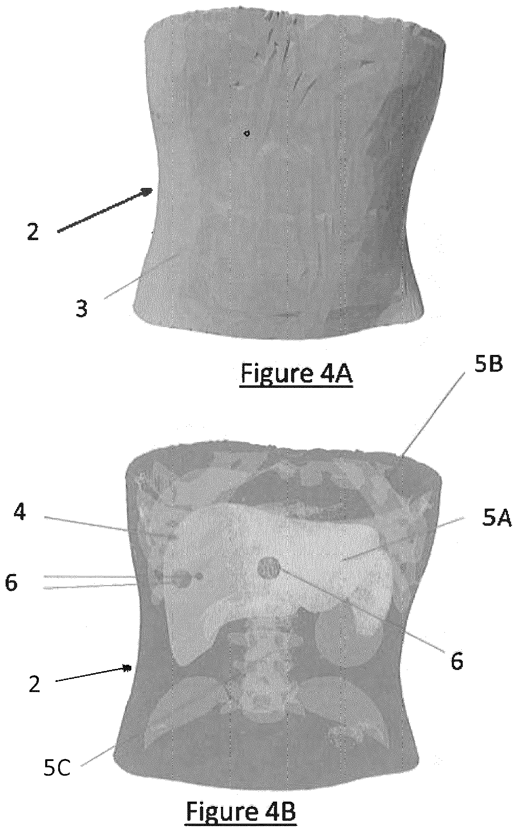

Il a été représenté sur les figures 4 une application du dispositif médical selon l'invention.

La figure 4A représente la vue externe réelle de l'abdomen du sujet.

Les figures 4B à 4E représentent la vue par l'opérateur (médecin ou autre..) porteur des lunettes/casque de réalité mixte de l'abdomen du sujet, qui voit le volume reconstruit en 3D projeté et fusionné avec et sur le volume réel.

La figure 4B représente la vue de l'abdomen du sujet et de son volume reconstruit (visualisation du foie 5A, de trois nodules hépatiques 6 dont un nodule cible, des côtes 5B et du rachis 5C obtenue après traitement d'images DICOM de son scanner abdominal) par réalité mixte au travers de lunettes/casque.

La figure 4C représente la vue en réalité mixte du sujet (réel et virtuel) avec l'aiguille et son pendant virtuel, l'émetteur et le récepteur.

La figure 4D représente la progression de l'aiguille 1 vers la cible avec un trajet ascendant, antéro-postérieur, sous costal (pour éviter la plèvre). L'aiguille 1 est à présent dans le foie 5A et est dirigée par l'opérateur en temps réel et en 3D jusqu'à sa cible.

La figure 4E représente l'aiguille 1 qui a atteint sa cible grâce au dispositif ;

La figure 4F représente la vision réelle (sans casque/lunettes) du sujet et de l'aiguille médicale 1A.

## Revendications

1. Dispositif médical de radiologie interventionnelle avec réalité mixte, permettant un guidage en temps réel et en 3D d'une aiguille médicale droite (1, 1A) pour réaliser une ponction, une biopsie, un drainage ou une ablation, vers au moins une cible (6) réelle dans un volume réel (3) de corps (2) d'un sujet,
comprenant :
- des moyens d'imagerie médicale (7a) ;
- un volume (4) reconstruit en 3D du volume réel (3), à partir d'images médicales préalablement acquises avant l'intervention et issues des moyens d'imagerie médicale (7a), incluant le volume (4) reconstruit en 3D avec des structures intérieures d'intérêt (5A, 5B, 5C) et une ou plusieurs cibles (6) reconstruites en 3D correspondant à la ou aux cibles (6) réelles ;
- des moyens de stockage (7b) du volume (4) reconstruit en 3D, pré-enregistré avant l'intervention, ces moyens de stockage (7b) étant reliés aux moyens d'imagerie médicale (7a) ;
- des moyens de visualisation (8) par réalité mixte pour visualiser le volume (4) reconstruit en 3D dans un référentiel virtuel de référence, destinés à être portés par un opérateur ; les moyens de visualisation (8) par réalité mixte communiquant avec des moyens de calcul (9) qui permettent de fusionner le volume (4) reconstruit en 3D avec le volume réel (3) ; le volume (4) reconstruit en 3D, une fois fusionné, demeurant stable et figé dans l'espace et dans le temps sur le volume réel (3), la cible (6) reconstruite 3D étant fusionnée avec la cible (6) réelle ;
- une aiguille médicale droite (1, 1A) destinée à être manipulée par l'opérateur dans la partie du corps (2) jusqu'à la cible (6) réelle ;
- des moyens (10A, 10B) de localisation et d'orientation en 3D, en temps réel, de l'aiguille médicale droite (1, 1A) dans la partie du corps (2), et reliés à l'aiguille médicale, fonctionnant par ondes électromagnétiques ;
- les moyens de calcul (9) communiquant avec les moyens d'imagerie médicale (7a), les moyens de stockage (7b), les moyens de visualisation (8), et les moyens (10A, 10B) de localisation et d'orientation en 3D, et recevant les coordonnées des moyens de visualisation (8) dans le repère réel, et le positionnement et l'orientation en 3D de l'aiguille médicale droite (1,1A) dans le référentiel réel,
les moyens de calcul (9) étant configurés pour :
i) déterminer les déplacements et rotations en temps réel des moyens de visualisation (8) dans le référentiel réel,
ii) déterminer la position du volume réel (3) dans le référentiel réel,
iii) créer une aiguille virtuelle droite (1,1B) fusionnée avec l'aiguille médicale (1,1A) réelle lors d'une étape de fusion, sur leur longueur, forme et positionnement, en tenant compte :
• du recalage en temps réel entre le référentiel virtuel de référence et le référentiel réel qui est fonction des déplacements et rotations en temps réel des moyens de visualisation (8) dans le référentiel réel,
• du positionnement de l'aiguille médicale droite (1,1A) dans le référentiel réel et transmis aux moyens de calcul (9), par les moyens (10A, 10B) de localisation et d'orientation en 3D,
• de la forme et de la longueur de l'aiguille médicale droite (1, 1A) intégrés dans les moyens de calcul (9),
afin de visualiser en temps réel à travers les moyens de visualisation (8), lors de l'intervention, le positionnement et le déplacement en 3D restant à parcourir jusqu'à la cible (6) reconstruite 3D de l'aiguille virtuelle droite (1,1B) dans le volume (4) reconstruit en 3D préenregistré ;
le positionnement virtuel et le déplacement virtuel fusionnant en temps réel à travers les moyens de visualisation (8), avec le positionnement réel et le déplacement en 3D réel de l'aiguille médicale droite (1, 1A) hors et dans le corps (2) du sujet,
pour guider en temps réel le déplacement par l'opérateur de l'aiguille médicale droite (1,1A) dans le volume réel (3) du corps (2) vers la cible (6) réelle, jusqu'à ce que l'extrémité de l'aiguille médicale droite (1,1A), atteigne la cible (6) réelle du volume réel (3),
les moyens (10A, 10B) de localisation et d'orientation en 3D, en temps réel, de l'aiguille médicale (1,1A) dans la partie du corps (2), présentant :
- au moins un capteur de position (10A) émettant des ondes électromagnétiques, ou respectivement un dispositif de réception (10B) des ondes électromagnétiques, situé sur l'aiguille médicale (1,1A) ;
- un dispositif de réception (10B) des ondes électromagnétiques, ou respectivement au moins un capteur de position (10A) émettant des ondes électromagnétiques, en dehors de l'aiguille médicale (1, 1A).

2. Dispositif médical selon la revendication 1, dans lequel les moyens de calcul (9) déterminent le recalage entre le référentiel virtuel de référence et le référentiel réel, en créant:
- un premier objet virtuel à sélectionner dans le référentiel virtuel de référence et dont les coordonnées réelles sont acquises dans le référentiel réel ;
- un second objet virtuel à sélectionner dans le référentiel virtuel de référence et dont les coordonnées réelles sont acquises dans le référentiel réel,
afin de calculer le décalage entre les deux référentiels,
pour faire fusionner en temps réel aiguille médicale (1,1A) et aiguille virtuelle (1,1B) lors du recalage correspondant à l'étape de fusion.

3. Dispositif médical selon la revendication précédente, dans lequel le dispositif médical comprend :
- les moyens d'imagerie (7a) qui sont aptes à réaliser des images en début ou en cours d'intervention de façon à permettre le recalage avec les images médicales préalablement acquises avant l'intervention et qui présente les volumes d'intérêts reconstruits (4), dont la ou les cibles.
- les moyens de calcul (9) qui sont aptes à calculer le recalage et de synchroniser en temps réel les référentiels virtuel et réel, par des repères virtuels choisis et dont les coordonnées réelles sont acquises dans le référentiel réel, par exemple parmi la liste suivante : des reliefs cutanés (ombilic, mamelons) ou osseux palpables (côtes, crêtes iliaques, épaule, sternum, épines vertébrales...), la surface de la peau, reconstruite en 3D à partir de l'imagerie médicale pré-opératoire.

4. Dispositif médical selon l'une des revendications précédentes, dans lequel les moyens de calcul (9) sont configurés pour réaliser un second repère virtuel fixe dans le référentiel virtuel de référence à proximité de l'aiguille virtuelle (1,1B) pour quantifier le déplacement de l'aiguille virtuelle (1,1B).

5. Dispositif médical selon l'une des revendications précédentes, dans lequel les images médicales utilisées sont de type DICOM et sont traitées par segmentation, seuillage, conversion en volume 3D par la technique de rendu de volume.

6. Dispositif médical selon l'une des revendications précédentes, dans lequel le volume (4) reconstruit en 3D présente une partie reconstruite comme la surface du sujet, des structures intérieures d'intérêt (5A, 5B, 5C) et la ou les cibles (6).

7. Dispositif médical selon l'une des revendications précédentes, dans lequel le dispositif médical comporte des seconds moyens de localisation et d'orientation en 3D en temps réel, de tout ou partie du sujet pour déterminer sa position dans le référentiel réel et donc les coordonnées réelles des repères choisis, et les moyens de calcul (9) communiquent avec ces seconds moyens, et sont configurés pour recalculer en continu le recalage entre les référentiels virtuel et réel et faire fusionner les mouvements de l'aiguille droite réelle (1, 1A) à ceux de l'aiguille droite virtuelle (1, 1B).

8. Dispositif médical selon l'une des revendications précédentes, dans lequel l'aiguille virtuelle (1,1B) présente des repères visibles permettant d'indiquer la longueur d'aiguille virtuelle (1,1B).

9. Dispositif médical selon l'une des revendications précédentes, dans lequel l'aiguille virtuelle (1,1B) présente des repères visibles comme une échelle avec des segments (13A, 13B) permettant d'indiquer la longueur d'aiguille virtuelle (1,1B).

10. Dispositif médical selon l'une des revendications précédentes, dans lequel l'aiguille virtuelle (1,1B) est composée de 2 éléments :
- la base, qui représente la position du capteur (10A) par rapport à l'émetteur (10B) dans l'espace des moyens de visualisation ;
- le corps multicolore représente le corps de l'aiguille.

11. Dispositif médical selon l'une des revendications précédentes, dans lequel l'aiguille virtuelle (1,1B) présente uniquement un contour destiné à coïncider avec le contour de l'aiguille réelle (1, 1A) dans la réalité mixte.

12. Dispositif médical selon l'une des revendications précédentes, dans lequel le volume réel (3) de corps (2) du sujet est une partie du corps (2) du sujet.

13. Dispositif médical selon l'une des revendications précédentes, dans lequel le volume réel (3) de corps (2) du sujet est l'ensemble du corps (2) du sujet.

14. Dispositif médical selon l'une des revendications précédentes, dans lequel les moyens d'imagerie (7a) sont choisis parmi la liste suivante : scanner, IRM, CBCT, échographie, Pet scanner ou Pet-IRM.

15. Dispositif médical selon l'une des revendications précédentes, dans lequel les moyens de calcul (9) sont reliés à des moyens de signalisation sonore pour indiquer par un signal sonore la distance qui sépare l'aiguille médicale droite (1,1A) des structures intérieures d'intérêt (5A, 5B, 5C) et des cibles (6).

## Patentansprüche

1. Medizinische Vorrichtung zum Einsatz in der interventionellen Radiologie mit Mischrealität, die eine 3D- und Echtzeitführung einer geraden medizinischen Nadel (1, 1A) zur Durchführung einer Punktion, einer Biopsie, einer Drainage oder einer Ablation zu wenigstens einem realen Ziel (6) in einem realen Volumen (3) eines Körpers (2) eines Probanden ermöglicht, umfassend:
- medizinische Bildgebungsmittel (7a);
- ein in 3D rekonstruiertes Volumen (4) des realen Volumens (3) aus medizinischen Bildern, die vor dem Eingriff erfasst wurden und aus medizinischen Bildgebungsmitteln (7a) stammen, die das in 3D rekonstruierte Volumen (4) mit interessierenden Innenstrukturen (5A, 5B, 5C) und ein oder mehrere in 3D rekonstruierte Ziele (6), die der oder den realen Zielmarke(n) (6) entsprechen, einschließen;
- Speichermedien (7b) des in 3D rekonstruierten, vor dem Eingriff voraufgezeichneten Volumens (4), wobei diese Speichermedien (7b) mit den medizinischen Bildgebungsmitteln (7a) verbunden sind;
- Visualisierungsmittel (8) durch Mischrealität zur Visualisierung des in 3D rekonstruierten Volumens (4) in einem virtuellen Referenz-Bezugssystem, die bestimmt sind, von einem Bediener getragen zu werden; wobei die Visualisierungsmittel (8) durch Mischrealität mit Berechnungsmitteln (9) kommunizieren, die es ermöglichen, das in 3D rekonstruierte Volumen (4) mit dem realen Volumen (3) zu verschmelzen; wobei das in 3D rekonstruierte Volumen (4), nachdem es verschmolzen wurde, räumlich und zeitlich auf dem realen Volumen (3) stabil und fixiert bleibt, wobei das rekonstruierte 3D-Ziel (6) mit dem realen Ziel (6) verschmolzen ist;
- eine gerade medizinische Nadel (1, 1A), die bestimmt ist, von dem Bediener in dem Teil des Körpers (2) bis zum realen Ziel (6) bedient zu werden;
- Mittel (10A, 10B) zur Lokalisierung und Orientierung der geraden medizinischen Nadel (1, 1A) in dem Teil des Körpers (2) in Echtzeit in 3D und verbunden mit der medizinischen Nadel, die mit elektromagnetischen Wellen betrieben werden;
- die Berechnungsmittel (9), die mit den medizinischen Bildgebungsmitteln (7a), den Speichermedien (7b), den Visualisierungsmitteln (8) und den Mitteln (10A, 10B) zur Lokalisierung und Orientierung in 3D kommunizieren und die Koordinaten der Visualisierungsmittel (8) im realen Bezugssystem sowie die 3D-Positionierung und - Orientierung der geraden medizinischen Nadel (1, 1A) im realen Bezugssystem empfangen,
wobei die Berechnungsmittel (9) ausgelegt sind, um:
i) die Verlagerungen und Rotationen der Visualisierungsmittel (8) im realen Bezugssystem in Echtzeit zu bestimmen,
ii) die Position des realen Volumens (3) im realen Bezugssystem zu bestimmen,
iii) eine virtuelle gerade Nadel (1, 1B) zu erzeugen, die in einem Verschmelzungsschritt mit der realen medizinischen Nadel (1, 1A) über ihre Länge, Form und Positionierung verschmolzen ist unter Berücksichtigung:
• der Neusynchronisierung in Echtzeit zwischen dem virtuellen Referenz-Bezugssystem und dem realen Bezugssystem, die von den Verlagerungen und Rotationen der Visualisierungsmittel (8) im realen Bezugssystem in Echtzeit abhängt,
• der Positionierung der geraden medizinischen Nadel (1, 1A) im realen Bezugssystem und übertragen an die Berechnungsmittel (9) von den Mitteln (10A, 10B) zur Lokalisierung und Orientierung in 3D,
• der in den Rechenmitteln (9) integrierten Form und Länge der geraden medizinischen Nadel (1, 1A),
um während des Eingriffs in Echtzeit anhand der Visualisierungsmittel (8) die Positionierung und die Verlagerung in 3D zu visualisieren, die noch von der geraden virtuellen Nadel (1, 1B) im zuvor aufgezeichneten, in 3D rekonstruierten Volumen (4) bis zum rekonstruierten 3D-Ziel (6) zurückzulegen ist;
wobei die virtuelle Positionierung und die virtuelle Verlagerung in Echtzeit anhand der Visualisierungsmittel (8) mit der realen Positionierung und der realen Verlagerung in 3D der geraden medizinischen Nadel (1, 1A) außerhalb und im Körper (2) des Probanden verschmelzen,
um die Verlagerung der geraden medizinischen Nadel (1, 1A) durch den Bediener im realen Volumen (3) des Körpers (2) in Echtzeit zum realen Ziel (6) zu führen, bis das Ende der geraden medizinischen Nadel (1, 1A) das reale Ziel (6) des realen Volumens (3) erreicht,
wobei die Mittel (10A, 10B) zur Lokalisierung und Orientierung in 3D der medizinischen Nadel (1, 1A) in Echtzeit im Teil des Körpers (2) aufweisen:
- wenigstens einen Positionssensor (10A), der elektromagnetische Wellen sendet oder bzw. eine Empfangsvorrichtung (10B) der elektromagnetischen Wellen, die sich auf der medizinischen Nadel (1, 1A) befindet;
- eine Empfangsvorrichtung (10B) der elektromagnetischen Wellen oder bzw. wenigstens einen Positionssensor (10A), der elektromagnetische Wellen sendet, außerhalb der medizinischen Nadel (1, 1A).

2. Medizinische Vorrichtung nach Anspruch 1, wobei die Berechnungsmittel (9) die Neusynchronisierung zwischen dem virtuellen Referenz-Bezugssystem und dem realen Bezugssystem bestimmen, indem sie erzeugen:
- ein erstes virtuelles Objekt, das aus dem virtuellen Referenz-Bezugssystem ausgewählt wird und dessen reale Koordinaten im realen Bezugssystem erfasst werden;
- ein zweites virtuelles Objekt, das aus dem virtuellen Referenz-Bezugssystem ausgewählt wird und dessen reale Koordinaten im realen Bezugssystem erfasst werden;
um den Versatz zwischen den zwei Bezugselementen zu berechnen,
um die medizinische Nadel (1, 1A) und die virtuelle Nadel (1, 1B) bei der Neusynchronisierung, die dem Verschmelzungsschritt entspricht, in Echtzeit zu verschmelzen.

3. Medizinische Vorrichtung nach vorstehendem Anspruch, wobei die medizinische Vorrichtung umfasst:
- Bildgebungsmittel (7a), die in der Lage sind, Bilder zu Beginn oder während des Eingriffs zu erstellen, um die Neusynchronisierung mit den vor dem Eingriff erfassten medizinischen Bildern zu ermöglichen und die die rekonstruierten interessierenden Volumen (4) einschließlich des/der Ziels/Ziele, darstellt,
- Berechnungsmittel (9), die in der Lage sind, die Neusynchronisierung zu berechnen und das virtuelle und reale Bezugssystem anhand ausgewählter virtueller Bezugspunkte in Echtzeit zu synchronisieren und deren reale Koordinaten im realen Bezugssystem erfasst werden, z. B. aus der folgenden Liste: Hautreliefs (Nabel, Brustwarze) oder tastbare Knochenreliefs (Rippen, Beckenkamm, Schulter, Sternum, Dornfortsätze...), Hautoberfläche, rekonstruiert in 3D ausgehend von der präoperativen medizinischen Bildgebung.

4. Medizinische Vorrichtung nach einem der vorhergehenden Ansprüche, wobei die Berechnungsmittel (9) ausgelegt sind, um einen zweite festen virtuellen Bezugspunkt im virtuellen Referenz-Bezugssystem in der Nähe der virtuellen Nadel (1, 1B) zur Quantifizierung der Verlagerung der virtuellen Nadel (1, 1B) herzustellen.

5. Medizinische Vorrichtung nach einem der vorhergehenden Ansprüche, wobei die verwendeten medizinischen Bilder vom Typ DICOM sind und durch Segmentierung, Schwellwertbestimmung, Umwandlung in ein 3D-Volumen durch Volumenwiedergabetechnik verarbeitet werden.

6. Medizinische Vorrichtung nach einem der vorhergehenden Ansprüche, wobei das in 3D rekonstruierte Volumen (4) einen rekonstruierten Teil wie die Oberfläche des Probanden, interessierende Innenstrukturen (5A, 5B, 5C) und das/die Ziel(e) (6) aufweist.

7. Medizinische Vorrichtung nach einem der vorhergehenden Ansprüche, wobei die medizinische Vorrichtung zweite Mittel zur Lokalisierung und Orientierung in 3D des gesamten oder eines Teils des Probanden in Echtzeit umfasst, um seine Position im realen Bezugssystem und damit die realen Koordinaten der gewählten Bezugspunkte zu bestimmen und die Berechnungsmittel (9) mit diesen zweiten Mitteln kommunizieren und ausgelegt sind, um kontinuierlich die Neusynchronisierung zwischen dem virtuellen und dem realen Bezugssystem neu zu berechnen und die Bewegungen der realen geraden Nadel (1, 1A) mit denen der virtuellen geraden Nadel (1, 1B) zu verschmelzen.

8. Medizinische Vorrichtung nach einem der vorhergehenden Ansprüche, wobei die virtuelle Nadel (1,1B) sichtbare Bezugspunkte aufweist, die erlauben, die Länge der virtuellen Nadel (1, 1B) anzugeben.

9. Medizinische Vorrichtung nach einem der vorhergehenden Ansprüche, wobei die virtuelle Nadel (1, 1B) sichtbare Bezugspunkte wie eine Skala mit Segmenten (13A, 13B) aufweist, die erlauben, die Länge der virtuellen Nadel (1, 1B) anzugeben.

10. Medizinische Vorrichtung nach einem der vorhergehenden Ansprüche, wobei die virtuelle Nadel (1, 1B) aus zwei Elementen zusammengesetzt ist:
- der Basis, die die Position des Sensors (10A) in Bezug auf den Sender (10B) im Raum der Visualisierungsmittel darstellt;
- dem mehrfarbigen Körper, der den Nadelkörper darstellt.

11. Medizinische Vorrichtung nach einem der vorhergehenden Ansprüche, wobei die virtuelle Nadel (1, 1B) nur eine Kontur aufweist, die bestimmt ist, in der Mischrealität mit der Kontur der realen Nadel (1, 1A) übereinzustimmen.

12. Medizinische Vorrichtung nach einem der vorhergehenden Ansprüche, wobei das reale Volumen (3) des Körpers (2) des Probanden ein Teil des Körpers (2) des Probanden ist.

13. Medizinische Vorrichtung nach einem der vorhergehenden Ansprüche, wobei das reale Volumen (3) des Körpers (2) des Probanden der gesamte Körper (2) des Probanden ist.

14. Medizinische Vorrichtung nach einem der vorhergehenden Ansprüche, wobei die Bildgebungsmittel (7a) aus der folgenden Liste ausgewählt sind: CT, MRT, CBCT, Ultraschall, Pet CT oder Pet-MRT.

15. Medizinische Vorrichtung nach einem der vorhergehenden Ansprüche, wobei die Rechenmittel (9) mit akustischen Signalmitteln verbunden sind, um durch ein akustisches Signal den Abstand anzugeben, der die gerade medizinische Nadel (1, 1A) von den interessierenden inneren Strukturen (5A, 5B, 5C) und den Zielen (6) trennt.

## Claims

1. An interventional radiology medical device with mixed reality, enabling real-time and 3D guidance of a straight medical needle (1,1A) to perform a puncture, biopsy, drainage or ablation, to at least one real target (6) in a real volume (3) of a subject's body (2), comprising:
- medical imaging means (7a);
- a 3D reconstructed volume (4) of the real volume (3), from medical images previously acquired before the intervention and derived from medical imaging means (7a), including the 3D reconstructed volume (4) with internal structures of interest (5A, 5B, 5C) and one or more 3D reconstructed targets (6) corresponding to the real target(s) (6);
- storage means (7b) for storing the 3D reconstructed volume (4), prerecorded before the intervention, these storage means (7b) being connected to the medical imaging means (7a);
- viewing means (8) per mixed reality to view the 3D reconstructed volume (4) in a virtual reference frame, for being carried by an operator; the viewing means (8) per mixed reality communicating with calculation means (9) allowing to merge the 3D reconstructed volume (4) with the real volume (3); the 3D reconstructed volume (4), once merged, remaining stable and frozen in space and over time on the real volume (3), the 3D reconstructed target (6) being merged with the real target (6);
- a straight medical needle (1,1 A) to be handled by the operator in the body part (2) up to the real target (6);
- means (10A, 10B) for locating and 3D orienting, in real time, the straight medical needle (1,1A) in the body part (2), and connected to the medical needle, operating with electromagnetic waves;
- the calculation means (9) communicating with the medical imaging means (7a), the storage means (7b), the viewing means (8), and the locating and 3D orienting means (10A, 10B), and receiving the coordinates of the viewing means (8) in the real reference frame, and the 3D positioning and orientation of the straight medical needle (1,1A) in the real reference frame,
the calculation means (9) being configured to:
i) determine real-time displacements and rotations of the viewing means (8) in the real reference frame,
ii) determine the position of the real volume (3) in the real reference frame,
iii) create a straight virtual needle (1,1B) merged with the real medical needle (1,1A) in a merging step, along their length, shape and positioning, taking account of;
• the real-time resetting between the virtual reference frame and the real reference frame, which depends on the real-time movements and rotations of the viewing means (8) in the real reference frame,
• the positioning of the straight medical needle (1,1A) in the real reference frame and transmitted to the calculation means (9), by the locating and 3D orienting means (10A, 10B),
• the shape and length of the straight medical needle (1,1A) integrated into the calculation means (9),
in order to view in real time through the viewing means (8), during the intervention, the positioning and 3D movement remaining to be travelled to the 3D reconstructed target (6) of the straight virtual needle (1,1B) in the 3D reconstructed volume (4) prerecorded;
the virtual positioning and virtual displacement merging in real time through the viewing means (8), with the real positioning and real 3D movement of the straight medical needle (1,1A) out of and into the subject's body (2).
to guide in real time the movement by the operator of the straight medical needle (1,1A) in the real volume (3) of the body (2) to the real target (6), until the end of the straight medical needle (1,1A) reaches the real target (6) of the real volume (3),
the locating and 3D orienting means (10A, 10B), in real-time, of the medical needle (1,1A) in the body part (2), having:
- at least one position sensor (10A) emitting electromagnetic waves, or respectively a device for receiving (10B) electromagnetic waves, situated on the medical needle (1,1A);
- a device for receiving (10B) electromagnetic waves, or respectively at least one position sensor (10A) emitting electromagnetic waves, outside the medical needle (1,1A).

2. The medical device according to claim 1, wherein the calculation means (9) determine resetting between the virtual reference frame and the real reference frame, by creating:
- a first virtual object to be selected in the reference virtual reference frame and whose real coordinates are acquired in the real reference frame;
- a second virtual object to be selected in the reference virtual reference frame and whose real coordinates are acquired in the real reference frame,
in order to calculate the offset between both reference frames,
to merge in real time medical needle (1,1A) and virtual needle (1,1B) upon resetting corresponding to the merging step.

3. The medical device according to the preceding claim, wherein the medical device comprises:
- the imaging means (7a) which are capable of producing images at the beginning or during the intervention so as to allow resetting with the medical images previously acquired before the intervention and which have the reconstructed volumes of interest (4), including the target(s).
- the calculation means (9) which are able to calculate resetting and synchronise in real time the virtual and real reference frames, by virtual markers chosen and whose real coordinates are acquired in the real reference frame, for example from the following list; skin reliefs (umbilic, nipples) or palpable bones (ribs, iliac crests, shoulder, sternum, vertebral spines...), the surface of the skin, 3D reconstructed from pre-operative medical imaging.

4. The medical device according to one of the preceding claims, wherein the calculation means (9) are configured to make a second fixed virtual marker in the virtual reference frame in proximity to the virtual needle (1,1B) to quantify movement of the virtual needle (1,1B).

5. The medical device according to one of the preceding claims, wherein the medical images used are of DICOM-type and are processed by segmentation, thresholding, conversion to 3D volume by the volume rendering technique.

6. The medical device according to one of the preceding claims, wherein the 3D reconstructed volume (4) has a part reconstructed as the surface of the subject, the internal structures of interest (5A, 5B, 5C) and the target(s) (6).

7. The medical device according to one of the preceding claims, wherein the medical device comprises second means for locating and 3D orienting in real-time all or part of the subject to determine its position in the real reference frame and thus the real coordinates of the chosen markers, and the calculation means (9) communicate with these second means, and are configured to continuously recalculate resetting between the virtual and real reference frames and merge the movements of the real straight needle (1,1A) with those of the virtual straight needle (1,1B).

8. The medical device according to one of the preceding claims, wherein the virtual needle (1,1B) has visible markers for indicating the length of the virtual needle (1,1B).

9. The medical device according to one of the preceding claims, wherein the virtual needle (1,1B) has visible markers as a scale with segments (13A, 13B) for indicating the length of the virtual needle (1,1B).

10. The medical device according to one of the preceding claims, wherein the virtual needle (1,1B) is comprised of 2 elements:
- the base, which represents the position of the sensor (10A) in relation to the transmitter (10B) in the viewing means space;
- the multi-coloured body represents the needle body.

11. The medical device according to one of the preceding claims, wherein the virtual needle (1,1B) has only a contour intended to coincide with the contour of the real needle (1,1A) in mixed reality.

12. The medical device according to one of the preceding claims, wherein the real volume (3) of the subject's body (2) is a part of the subject's body (2).

13. The medical device according to one of the preceding claims, wherein the real volume (3) of the subject's body (2) is the whole subject's body (2).

14. The medical device according to one of the preceding claims, wherein the imaging means (7a) are selected from the following list: scanner, MRI, CBCT, ultrasound, PET CT or PET-MRI.

15. The medical device according to one of the preceding claims, wherein the calculation means (9) are connected to audible signalling means to indicate by an audible signal the distance that separates the straight medical needle (1,1A) and the internal structures of interest (5A, 5B, 5C) and the targets (6).
